# EUROPEAN PATENT SPECIFICATION

(11) **EP 2 027 771 B1**
(45) Date of publication and mention of the grant of the patent: **13.06.2012**
(21) Application number: 07016663.2
(22) Date of filing: 24.08.2007
(51) Int. Cl.: A01K 67/027, A01K 67/02, C12Q 1/68

(54) **Sequencing the mitochondrial DNA with reference to the fertility as a means for the optimization of sow breeding lines**
Sequenzierung der mitochondrialen DNA in Bezug auf die Fortpflanzungsfähigkeit als Mittel zur Optimierung von Muttersau-Zuchtlinien
Séquençage de l'ADN mitochondrial par référence avec la fertilité comme moyen d'optimisation de lignées d'élevage de truies

(43) Date of publication of application: 25.02.2009
(73) Proprietor: Hermitage Pedigree Pigs Ltd., Kilkenny (IE)
(72) Inventor: Peeters, Ivar B., 5986 BK Beringe (NL); Olek, Klaus, 53115 Bonn (DE); Cors, Nicolas, 8712 Stäfa (CH); Jansen, Thomas, 50389 Wesseling (DE); Nolan, Edward, Kilkenny, Co. Kilkenny (IE); Murphy, Ronan, Tinahely, Co. Wicklow (IE); McGee, Robert, Kilkenny, Co. Kilkenny (IE)
(74) Representative: Krauss, Jan

(56) References cited:
- WO-A-00/36143
- WO-A-2004/063386
- WO-A-2005/073408
- US-A- 5 550 024
- YEN N-T; LIN C-S; JU C-C; WANG S-C; HUANG M-C: "Mitochondrial DNA polymorphism and determination of effects on reproductive trait in pigs" REPRODUCTION IN DOMESTIC ANIMALS, vol. 42, no. 4, 16 July 2007 (2007-07-16), - August 2007 (2007-08) pages 387-392, XP002465128
- .ALVES E, OVILO C, RODRÍGUEZ MC, SILIÓ L.: "Mitochondrial DNA sequence variation and phylogenetic relationships among Iberian pigs and other domestic and wild pig populations" ANIM GENET., vol. 34, no. 5, October 2003 (2003-10), pages 319-324, XP002484641
- URSING BM, ARNASON U.: "The complete mitochondrial DNA sequence of the pig (Sus scrofa)." THE COMPLETE MITOCHONDRIAL DNA SEQUENCE OF THE PIG (SUS SCROFA)., vol. 47, no. 3, September 1998 (1998-09), pages 302-306, XP002484642
- LIN ET AL.: "Complete nucleotide sequence of pig ( Sus scrofa) mitochondrial genome and dating evolutionary divergence within Artiodactyla" GENE, vol. 236, 1999, pages 107-114, XP002484643

## Description

The present invention relates to a method for optimizing sow breeding lines in terms of the breeding performance of sows by selecting pigs for breeding based on maternal fertility parameters indicated by the haplotype of their mitochondrial DNA (mtDNA) according to the method of independent claim 1.

There are approximately 5 million pigs in the UK, of which approximately 500,000 (10%) are used for breeding. The most common breeds of pigs used are the Landrace and Large White. The majority of pigs reared for meat in the UK are crossbreeds. For example, when a (Landrace x Large White) female or 'F1' is bred with a Terminal Sire, the resulting progeny are produced for slaughter. The terminal sirelines are specifically selected for terminal traits of economic importance such as growth rate, lean meat %, low backfat levels etc. The combination of the prolific maternal lines and the high yielding slaughter lines is specifically designed to maximize the economic returns to both the farmer and the processor.

Sows are first mated when they are 7-9 months old. Pregnancy lasts approximately 114 days and a sow can produce up to 25 piglets in a litter (averaging 10 - 13). The average number of pigs reared and sold per sow is 22 each year, though many sows rear more than this and top performing herds can sell up to 28 - 30 pigs per sow per year. The average number of parities of a sow is 4-7, before the sow is replaced.

Modern pig production, involves optimizing the performance of both the maternal and terminal lines to ensure maximum productivity and subsequent economic returns from the enterprise. Over the years, genetic advances have contributed significantly to improving the performance of the commercial breeding herd and the characteristics of the slaughter pig produced. Two markers in particular, the so-called PSS- and Napole gene (former: Porcine Stress Syndrome, mutation on the Ryanodine-receptor gene; latter: Rendement Napole, mutation on the γ-subunit of the AMP-activated protein kinase, PRKAG3), have been successfully utilized to eliminate deleterious meat quality traits and improve carcass traits of pig meat worldwide.

The use of Marker Assisted Selection, combined with the performance and sib test data, allows pig breeders to identify animals which have inherited desirable genes and thereby increase selection accuracy and speed up genetic progress even further.

The mitochondrial genome of the pig (Sus scrofa) is around 16620 base pairs in size (Lin C.S. et al., Gene 236 (1999) 107-114, DNA sequence was deposited to Genbank, Acc. No.: NC_000845 and AF034253). It is exclusively transmitted through the maternal line and comprises genes coding for rRNAs and tRNAs responsible for the replication of the mitochondrial DNA as well as genes coding for the essential protein components of the mitochondrial enzymatic apparatus, in particular the respiratory chain. The enzymes of the respiratory chain are embedded in the inner mitochondrial membrane and are essential to the process of oxidative phosphorylation, which generates most of the animal cell's energy in form of ATP.

Some tissues and metabolic networks and processes need a notably high amount of energy. Therefore, they are particularly susceptible to pathogenic mutations in the mitochondrial genome. Accordingly, it is likely that non-pathogenic variants as well influence processes demanding high energy inputs in manifold ways.

The publication of Yen N-T et al discloses the use of mitochondrial haplotypes present in maternal porcine lines. The authors investigate the association of single strand conformation polymorphisms (SSCP) in the D-loop region of the mitochondrial DNA with pig reproductive traits. The study revealed that a significant association is only found for the average body weight of piglets at day 21. For other maternal fertility parameters no association was observed (N-T Yen et al. 2007, Reprod Dom Anim 42, 387-392).

It is therefore an objective of the present invention to provide for an easy, fast, and cost efficient method to optimize sow breeding lines in terms of the breeding performance of sows by selecting genetically superior pigs for breeding. It was also an objective of the present invention to allow for a selection based on specific maternal fertility parameters. Furthermore, it was an objective of the present invention to correlate said maternal fertility parameters to specific genetic markers.

The objectives of the present invention are solved by a method for optimizing sow breeding lines in terms of an increased number of piglets born alive per sow and/or per year according to independent claim 1.

In one preferred embodiment said haplotype is defined by partial or complete sequencing of the mitochondrial genome.

Fertility parameters as described herein are selected from the group comprising number of piglets born alive, number of piglets born dead, and time period between weaning and subsequent mating.

The present disclosure describes a haplotype, which is characterized by the presence of polymorphisms, said polymorphisms comprising an adenine at position 692, a guanine at position 5392, an adenine at position 14233, a thymine at position 15695. Further described is a haplotype that comprises all these polymorphisms.

The numbering of the respective positions of the polymorphisms as mentioned is defined through the mtDNA sequence of the pig deposited by Lin et al. to Genbank, acc. no.: AF034253.

The present disclosure describes a haplotype, which is characterized by the presence of polymorphisms, said polymorphisms comprising a cytosine at position 560, a cytosine at position 1089, a thymine at position 5314, a cytosine at position 5356, an adenine at position 6762, a thymine at position 7230, a thymine at position 7389, a guanine at position 7461, an adenine at position 7536, a guanine at position 8607, a thymine at position 9951, a cytosine at position 11460, a thymine at position 11473, a thymine at position 15075, a thymine at position 15557, an adenine at position 15773, and a guanine at position 16475, wherein said haplotype is indicative of an decreased number of piglets born alive and of a reduced time period between two litters of one and the same sow. Further described is a haplotype that comprises all these polymorphisms.

The present disclosure describes a haplotype, which is characterized by the presence of polymorphisms, said polymorphisms comprising an adenine at position 124, a thymine at position 279, a thymine at position 323, an adenine at position 692, a cytosine at position 14706, and a thymine at position 15060. Further described is a haplotype that comprises all these polymorphisms.

The present disclosure describes a haplotype, which is characterized by the presence of polymorphisms, said polymorphisms comprising a cytosine at position 405, a cytosine at position 3492, a cytosine at position 4392, an adenine at position 6738, and a cytosine at position 15527, wherein said haplotype is indicative of a decreased number of piglets born alive. Further described is a haplotype that comprises all these polymorphisms.

The present disclosure describes a haplotype, which is characterized by the presence of polymorphisms, said polymorphisms comprising a cytosine at position 12496 and a cytosine at position 14471. Further described is a haplotype that comprises all these polymorphisms.

The present disclosure describes a haplotype, which is characterized by the presence of polymorphisms, said polymorphisms comprising an adenine at position 124, a guanine at position 704, a guanine at position 706, a thymine at position 2222, an adenine at position 5726, an adenine at position 5869, a guanine at position 7270, a cytosine at position 9173, a cytosine at position 9526, an adenine at position 9758, a thymine at position 10286, a cytosine at position 13999, an adenine at position 14020, and an adenine at position 15896. Further described is a haplotype that comprises all these polymorphisms.

The present disclosure describes a haplotype, which is characterized by the presence of polymorphisms, said polymorphisms comprising a thymine at position 2294, a cytosine at position 6219, a cytosine at position 9289, and an adenine at position 14218, wherein said haplotype is indicative of an increased number of piglets born dead. Further described is a haplotype that comprises all these polymorphisms.

In one preferred embodiment said haplotype is characterized by the presence of all the polymorphisms comprising compared to SEQ ID No. 1 a cytosine at position 560, a thymine at position 1089, a thymine at position 5314, a cytosine at position 5356, an adenine at position 6762, a thymine at position 7230, a thymine at position 7389, a guanine at position 7461, an adenine at position 7536, a guanine at position 8607, a thymine at position 9951, a cytosine at position 11460, a thymine at position 11473, a thymine at position 15075, a thymine at position 15557, an adenine at position 15773, and a guanine at position 16475, wherein said haplotype is indicative of an increased number of piglets born alive.

In one further preferred embodiment said haplotype is characterized by the presence of all the polymorphisms comprising compared to SEQ ID No. 1 a cytosine at position 405, an adenine at position 1234, a cytosine at position 3355, a thymine at position 4290, an adenine at position 8188, a thymine at position 8466, a guanine at position 9293, a cytosine at position 13393, and an adenine at position 16224, wherein said haplotype is indicative of an increased number of piglets born alive.

The present disclosure describes a haplotype, which is characterized by the presence of polymorphisms, said polymorphisms comprising a guanine at position 294, a thymine at position 657, an adenine at position 692, a cytosine at position 4392, a cytosine at position 9335, and a cytosine at position 13624, wherein said haplotype is indicative of an extended time period between two litters of one and the same sow. Further described is a haplotype that comprises all these polymorphisms.

In one further preferred embodiment said haplotype is characterized by the presence of all the polymorphisms comprising compared to SEQ ID No. 1 an adenine at position 124, a cytosine at position 214, a guanine at position 232, a thymine at position 692, a thymine at position 9348, and a cytosine at position 15644, wherein said haplotype is indicative of a decreased number of piglets born dead.

The term "optimizing the breeding performance" as used herein is meant to refer to an increased number of piglets born alive per sow and/or per year.

The term "haplotype" as used herein is meant to refer to a set or pattern of alleles of single nucleotide polymorphisms (SNPs) on the mitochondrial genome that are linearly linked, in particular statistically associated with the phenotypes as identified herein.

The term "correlating said haplotype with" as used herein is meant to refer to the statistical analysis of a group of pigs, having the same haplotype, in terms of a specific trait, in particular a specific maternal fertility parameter, in order to determine whether said haplotype is indicative of said specific trait.

The term "favourable haplotype for breeding" as used herein is meant to refer to haplotypes, which are indicative of an increased number of piglets born alive, a decreased number of piglets born dead, and a reduced time period between two litters of one and the same sow.

The term "maternal fertility parameter" as used herein is meant to refer to maternal traits that are suitable for describing the breeding performance of a sow.

The inventors have surprisingly found that maternal fertility parameters of pigs are significantly influenced by their mitochondrial haplotype. Therefore, precise examination of the mitochondrial DNA-sequence and its correlation to maternally inherited traits will allow the selection of genetically superior pigs for breeding and the optimization of sow breeding lines, particularly of those that are already among the top 5%. According to the present disclosure specific maternal fertility parameters, such as the number of piglets born alive/dead or the time period between two litters of one and the same sow, can be linked to specific haplotypes of the mtDNA. The partial or complete sequencing of the mtDNA of an individual pig, thereby defining its mitochondrial haplotype, followed by comparison to known haplotypes, for example to those disclosed herein, constitutes an easy, fast, and cost efficient method. The method of the present invention is suitable to test for the mitochondrial influence in any relevant breeding trait in pigs, and should therefore be of high economical value for pig breeding.

The invention is now further described by reference to the following examples which are intended to illustrate, not to limit the scope of the invention. In the Figures,

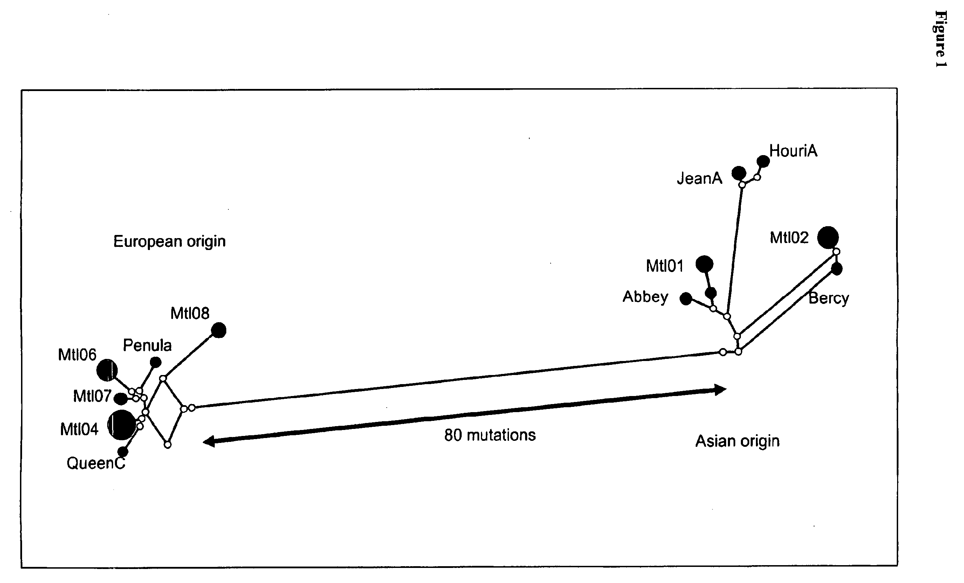

Figure 1 shows the phylogenetic tree calculated from the mitochondrial DNA sequences using Network 4.1.1.2 (www.fluxusengineering.com). The distances between the respective breeding lines are proportional to the number of polymorphisms. Empty circles depict so-called "Median vectors", filled, black circles depict existing haplotypes. The size of the black circles is proportional to the number of samples, which are represented by them.

### Example 1

### Sample Selection

69 sows related to different maternal lines were selected for testing. A tissue sample was withdrawn from every single pig. The animals descended from two different breeds ("Landrace" and "Large White").

**Table 1: List of the Landrace samples tested**

| **Line** | **Sow's ID** |
|---|---|
| Bodil | FVUM5483, FVUM8382, FVUP1245, FVUN0346, FVUK7193, FVUN6606 |
| Christina | FVUN0061 |
| Hajusinitti | FVUN5121, JAL0768, FVUN3580 |
| Kvam | LAL0385, FVUN4177, FVUK4875 |
| Nasia | FVUP0451, FVUN4262 |
| Penula | FAL0074, LAL0963 |
| Queen | FVUN5058, FVUP1514, FVUN4926, FVUN9694, FVUM9523, FVUL1962, FVUJ5486, FVUL5379, FVUN8824, FAL2534 |
| Ulpukka | FVUN7792, LAL0569 |

**Table 2: List of the Large White samples tested**

| **Line** | **Sow's ID** |
|---|---|
| Abbey | BCXH5211 |
| Arina | CAW0259 |
| Bercy | BCXG6863 |
| Champion Molly | BCXH1932, BCXG0239 |
| Dahila | BCXH2274, BCXH 1360 |
| Fanny | FAW0283, BCXG7042, CAW1232, BCXG6633 |
| Houri | BCXG2931, BCXF8684 |
| Jean | BCXF4883, BCXG9418 |
| Jetroko | BCXH1721, BCXG3409 |
| Maple Leaf | BCXH5289, BCXH1164 |
| Molly | BCXG1289, BCXF4530, FAW0191, FAW0239, BCXH2205, BCXG7358, BCXE0657, BCXG1162, FAW1215, BCXE4547, BCXG3731, FAY0637, BCXD9182, BCXF9562, BCXH6729 |
| Queen Mary | BCXG3836, BCXG2803, BCXG7053 |
| Royal Catalina | BCXH5051, BCXH5098 |
| Rouri | FAW0327 |

### Example 2

### Statistical methods

### Analysis of variance between groups (ANOVA)

In order to determine whether the mitochondrial genome influences particular breeding traits, an analysis of variance between groups (ANOVA) was performed. Even if there was not a "real" effect of the mitochondrial genome on the breeding value (the null hypothesis), the groups would still be likely to have different average breeding values. The expected range of variation of the averages in the case of the null hypothesis is given by the standard deviation of the estimated means: σ/*N*^{½}, wherein σ is the standard deviation of the breeding value of all the animals and *N* is the number of animals in a group.

**Definition** "breeding value": Genetically inherited value of a breeding animal, which refers to random mating within a population and relies on additive genetic effects.

The comparison between the actual variation of the group averages and that expected from the above formula is expressed by the *F* ratio:
*F* = (found variation of the group averages)/(expected variation of the group averages)
Thus if the null hypothesis is correct, *F* is expected to be about 1, whereas an *F* with a value >1 indicates a haplotype effect.

The significance of an *F* value >1 is determined by a probabilistic evaluation. The significance level is, depending on the model used, defined to be <0,05 or <0,01.

The maximal effect of the mitochondrial genome is represented by the adjusted R-square (R²_{adj}). R-Square (R²) is the proportion of variance in the dependent variable (breeding value) which can be predicted from the independent variables (mitochondrial haplotype). This is an overall measure of the strength of association, and does not reflect the extent to which any particular independent variable is associated with the dependent variable. R²_{adj} attempts to yield a more honest value to estimate the R² for the population and is calculated using the formula 1 - {(1 - R²) [(N - 1) / (N - k - 1)]}, wherein N is the number of observations and k the number of predictor.

### Principle of the test

Since fertility parameters are very complex phenotypes, the nuclear DNA will also have a significant influence. The influence of the nuclear genome takes effect both through the maternal and the paternal line. The different proportions in the inheritance of the fertility parameters were tested by 4 independent ANOVA analyses:
1. Paternal, nuclear encoded fraction of the inherited fertility
2. Maternal, nuclear encoded fraction of the inherited fertility
3. Mitochondrial fraction and fertility
4. Geographical origin and fertility

The variances for the four different parameters were estimated using the following bench marks:
- Parameter 1:: 813 pigs out of 30 boars
- Parameter 2:: 813 pigs out of 22 sows
- Parameter 3:: 813 pigs with 18 different mitochondrial DNA-sequences
- Parameter 4:: 813 pigs with 2 different origins (Europe or Asia)

The determinants were:
- Character 1:: Number of piglets born alive
- Character 2:: Number of piglets born dead
- Character 3:: Time between weaning and subsequent mating

R²_{adj} was calculated for the different parameters using the respective characters (Tables 4, 6, 8, summarized in Table 11).

### Example 3

### Phylogenetic and statistical analyses

It is important to differentiate the influence of the nuclear genome and exogenic factors (e.g. nutrition) from the influence of the mitochondrial genome. For the present study, data from 813 sows, which gave birth to 2740 litters (all related to the analyzed 69 samples), were collected and analyzed. Because of the fact that animals, which were definitely unrelated (in terms of their nuclear DNA), showed comparable breeding performances and similar sequence patterns on the mitochondrial DNA, it was possible to extract the mitochondrial influence from the other possible influences. Because the traits tested were not governed by the mitochondrial DNA alone, the effect of the nuclear encoded influence from the sire- and the dam-subline was estimated as well.

### Phylogenetic analysis of breeding lines based on their mitochondrial haplotypes

The complete mitochondrial genome of 69 sows was sequenced, leading to the identification of 13 different haplotypes comprising a total of 198 polymorphisms (mutations). The respective haplotypes were combined into clusters, which are based on a phylogenetic tree (Figure 1). These clusters, which have been composed out of several maternal lines with identical haplotype, were gathered to the groups listed in Table 3.

**Table 3: Listing of the haplotypes gathered to mitochondrial lines**

| **Groups** | **Haplotypes** |
|---|---|
| MtI01 | Fanny C, Arina, Dahila |
| MtI02 | Queen Mary B, Molly B, Maple Leaf A, Houri B, Fanny B, Jean B, Royal Catalina, Champion Molly, Jetroko A |
| MtI04 | Queen Mary A, Kvam, Bodil A, Queen B, Molly A, Fanny A, Hajusinitti B |
| MtI06 | Hajusinitti A, Bodil B, Christina, Nasia B, Queen A |
| MtI07 | NasiaA, Ulpukka |
| MtI08 | Jetroko B, Rouri |

The respective haplotypes, with the numbers being based on Lin,C.S. et al., *Sus scrofa* mitochondrion, complete genome, Genbank Acc. No.: C_000845, could be characterized as follows:
Mt101 (Asian origin): 692 A, 5392 G, 14233 A, 15695 T
Mt102 (Asian origin): 560 C, 1089 C, 5314 T, 5356 C, 6762 A, 7230 T, 7389 T, 7461 G, 7536 A, 8607 G, 9951 T, 11460 C, 11473 T, 15075 T, 15557 T, 15773 A, 16475 G
Mt104 (European origin): 124 A, 279 T, 323 T, 692 A, 14706 C, 15060 T
Mt106 (European origin): 405 C, 3492 C, 4392 C, 6738 A, 15527 C
Mt107 (European origin): 12496 C, 14471 C
Mt108 (European origin): 124 A, 704 G, 706 G, 2222 T, 5726 A, 5869 A, 7270 G, 9173 C, 9526 C, 9758 A, 10286 T, 13999 C, 14020 A, 15896 A
Abbey (Asian origin): 2294 T, 6219 C, 9289 C, 14218 A
Bercy (Asian origin): 560 C, 1089 T, 5314 T, 5356 C, 6762 A, 7230 T, 7389 T, 7461 G, 7536 A, 8607 G, 9951 T, 11460 C, 11473 T, 15075 T, 15557 T, 15773 A, 16475 G
Houri A (Asian origin): 405 C, 1234 A, 3355 C, 4290 T, 8188 A, 8466 T, 9293 G, 13393 C, 16224 A
Jean A (Asian origin): 405 C, 1234 A, 3355 C, 8188 A, 8466 T, 9293 G, 13393 C, 16224 A Penula (European origin): 294 G, 657 T, 692 A, 4392 C, 9335 C, 13624 C
Queen C (European origin): 124 A, 214 C, 232 G, 323 T, 692 A, 9348 T, 15644 C

### Statistical analysis of specific maternal fertility parameters

### Number of piglets born alive

**Table 4: The given values "maximal effect" display the maximal variance which can be traced back to the respective parameter - sire subline etc.**

| | |
|---|---|
| Maximal effect of the sire subline : | 6,5 % |
| Maximal effect of the dam subline : | 3,3 % |
| Maximal effect of the mitochondrial haplotype: | 1,0 % |
| I Maximal effect of origin (Asian or European): | not significant |

**Table 5: Comparison of all lines with reference to the average number of piglets born alive (pba)**

| **Level** | **Individuals** | **Mean no. pba** | **Std Error** | **Lower 95%** | **Upper 95%** |
|---|---|---|---|---|---|
| Abbey | 11 | 12,5455 | 0,60013 | 11,369 | 13,722 |
| Bercy | 10 | 14,2000 | 0,62942 | 12,966 | 15,434 |
| Houri-A | 10 | 13,6333 | 0,62942 | 12,399 | 14,868 |
| MtI01 | 229 | 11,8020 | 0,13153 | 11,544 | 12,060 |
| MtI02 | 359 | 11,5810 | 0,10505 | 11,375 | 11,787 |
| MtI04 | 1279 | 11,7457 | 0,05566 | 11,637 | 11,855 |
| MtI06 | 511 | 11,5326 | 0,08805 | 11,360 | 11,705 |
| MtI07 | 86 | 11,6693 | 0,21463 | 11,248 | 12,090 |
| MtI08 | 12 | 12,0833 | 0,57458 | 10,957 | 13,210 |
| Penula | 95 | 11,7737 | 0,20421 | 11,373 | 12,174 |
| Queen-C | 138 | 11,6611 | 0,16943 | 11,329 | 11,993 |

**Table 6: The given values "maximal effect" display the maximal variance which can be traced back to the respective parameter - sire subline etc.**

| Number of piglets born dead | |
|---|---|
| Maximal effect of the sire subline: | 8,3 % |
| Maximal effect of the dam subline: | 6,6 % |
| haplotype: Maximal effect of the mitochondrial haplotype: | 5,3 % |
| Maximal effect of origin (Asian or European): | 0,1 % |

**Table 7: Comparison of all lines with reference to the average number of piglets born dead (pbd)**

| **Level** | **Individuals** | **Mean no. pbd** | **Std Error** | **Lower 95%** | **Upper 95%** |
|---|---|---|---|---|---|
| Abbey | 11 | 1,18182 | 0,16495 | 0,8584 | 1,5053 |
| Bercy | 10 | 2,50000 | 0,17300 | 2,1608 | 2,8392 |
| Houri-A | 10 | 0,43333 | 0,17300 | 0,0941 | 0,7726 |
| MtI01 | 229 | 0,54642 | 0,03615 | 0,4755 | 0,6173 |
| MtI02 | 359 | 0,52414 | 0,02887 | 0,4675 | 0,5808 |
| MtI04 | 1279 | 0,49135 | 0,01530 | 0,4614 | 0,5213 |
| MtI06 | 511 | 0,56292 | 0,02420 | 0,5155 | 0,6104 |
| MtI07 | 86 | 0,66053 | 0,05899 | 0,5449 | 0,7762 |
| MtI08 | 12 | 0,58333 | 0,15793 | 0,2737 | 0,8930 |
| Penula | 95 | 0,65263 | 0,05613 | 0,5426 | 0,7627 |
| Queen-C | 138 | 0,47899 | 0,04657 | 0,3877 | 0,5703 |

**Table 8: The given values "maximal effect" display the maximal variance which can be traced back to the respective parameter - sire subline etc.**

| Time period between two litters | |
|---|---|
| Maximal effect of the sire subline: | 3,0 % |
| Maximal effect of the dam subline: | 2,0 % |
| Maximal effect of the mitochondrial Haplotype: | 1,2 % |
| Maximal effect of origin (Asian or European): | 0,2 % |

**Table 9: Comparison of all lines with reference to the average time period between two litters (time between weaning and subsequent mating}**

| **Level** | **Individuals** | **Mean time [months]** | **Std Error** | **Lower 95%** | **Upper 95%** |
|---|---|---|---|---|---|
| Abbey | 11 | 2,40798 | 0,04458 | 2,3206 | 2,4954 |
| Bercy | 10 | 2,40909 | 0,04676 | 2,3174 | 2,5008 |
| Houri-A | 10 | 2,40864 | 0,04676 | 2,3170 | 2,5003 |
| MtI01 | 229 | 2,44168 | 0,00977 | 2,4225 | 2,4608 |
| MtI02 | 359 | 2,40844 | 0,00780 | 2,3931 | 2,4237 |
| MtI04 | 1279 | 2,43031 | 0,00413 | 2,4222 | 2,4384 |
| MtI06 | 511 | 2,43017 | 0,00654 | 2,4173 | 2,4430 |
| MtI07 | 86 | 2,45974 | 0,01594 | 2,4285 | 2,4910 |
| MtI08 | 12 | 2,38201 | 0,04268 | 2,2983 | 2,4657 |
| Penula | 95 | 2,48408 | 0,01517 | 2,4543 | 2,5138 |
| Queen-C | 138 | 2,47322 | 0,01259 | 2,4485 | 2,4979 |

**Table 10: Calculated F values and the corresponding significances for the three characters tested**

| Summarizing tables | | |
|---|---|---|
| The fing values for the three analyzed breeding traits were found: | | |
| | *F* ratio | Prob. > *F* |
| Character 1 | 6,3217 | <0,0001 |
| Character 2 | 12,8086 | <0,0001 |
| Character 3 | 3,6173 | <0,0001 |

This calculation shows clearly that the mitochondrial genome has got a significant influence on the analyzed breeding traits. The probability that these variance variations arose just "by chance" is less that 1/10.000 (Prob > *F* = the significance of the result [also called p-value; the smaller the p-value, the more significant the result is said to be])

**Table 11: Summary of the R²_{adj}-values from tables 4, 6, and 8. n.s. = not significant**

| | Character | 1 | 2 | 3 |
|---|---|---|---|---|
| Parameter | | | | |
| 1 | | 1,0 | 5,3 | 1,2 |
| 2 | | 3,3 | 6,6 | 2,0 |
| 3 | | 6,5 | 8,3 | 3,0 |
| 4 | | n.s. | 0,1 | 0,2 |

## Claims

1. A method for optimizing sow breeding lines in terms of an increased number of piglets born alive per sow and/or per year, said method comprising the steps of:
a) defining the haplotypes of the mitochondrial genome from a group of pigs,
b) correlating said haplotypes with at least one maternal fertility parameter selected from the group consisting of the number of piglets born alive, the number of piglets born dead, and the time period between weaning and subsequent mating of one and the same sow, and
c) selecting the sow for breeding having a haplotype significantly correlated with at least one desired maternal fertility parameter selected from the group consisting of an increased number of piglets born alive, a reduced number of piglets born dead and a reduced time period between two litters of one and the same sow,
wherein said haplotype is **characterized by** the presence of all the polymorphisms comprising compared to SEQ ID No. 1 a cytosine at position 560, a thymine at position 1089, a thymine at position 5314, a cytosine at position 5356, an adenine at position 6762, a thymine at position 7230, a thymine at position 7389, a guanine at position 7461, an adenine at position 7536, a guanine at position 8607, a thymine at position 9951, a cytosine at position 11460, a thymine at position 11473, a thymine at position 15075, a thymine at position 15557, an adenine at position 15773, and a guanine at position 16475, wherein said haplotype is indicative of an increased number of piglets born alive ;
or wherein said haplotype is **characterized by** the presence of all the polymorphisms comprising compared to SEQ ID No. 1 a cytosine at position 405, an adenine at position 1234, a cytosine at position 3355, a thymine at position 4290, an adenine at position 8188, a thymine at position 8466, a guanine at position 9293, a cytosine at position 13393, and an adenine at position 16224, wherein said haplotype is indicative of an increased number of piglets born alive ;
or wherein said haplotype is **characterized by** the presence of all the polymorphisms comprising compared to SEQ ID No. 1 an adenine at position 124, a cytosine at position 214, a guanine at position 232, a thymine at position 692, a thymine at position 9348, and a cytosine at position 15644, wherein said haplotype is indicative of a decreased number of piglets born dead.

2. The method according to claim 1, wherein said haplotype is defined by partial or complete sequencing of the mitochondrial genome.

## Patentansprüche

1. Verfahren zur Optimierung von Sau-Zuchtlinien im Hinblick auf eine erhöhte Anzahl von lebend geborenen Ferkeln pro Sau und/oder pro Jahr, wobei das Verfahren die Schritte umfasst von:
a) Definieren der Haplotypen des mitochondriellen Genoms einer Gruppe von Schweinen,
b) Korrelieren der Haplotypen mit mindestens einem maternalen Fertilitätsparameter ausgewählt aus der Gruppe bestehend aus der Zahl von lebend geborenen Ferkeln, der Zahl von tot geborenen Ferkeln, und der time Zeitspanne zwischen der Entwöhnung und anschließender Paarung von ein und derselben Sau, und
c) Auswählen der Sau zum Züchten, die einen Haplotyp aufweist, der signifikant mit mindestens einem erwünschten matemalen Fertilitätsparameter ausgewählt aus der Gruppe bestehend aus einer an erhöhten Anzahl von lebend geborenen Ferkeln, einer verringerten Anzahl von tot geborenen Ferkeln und einer verringerten Zeitspanne zwischen zwei Würfen von ein und derselben Sau korreliert,
wobei der Haplotyp durch die Anwesenheit aller Polymorphismen **gekennzeichnet** ist, umfassend, verglichen mit SEQ ID Nr. 1, ein Cytosin an Position 560, ein Thymin an Position 1089, ein Thymin an Position 5314, ein Cytosin an Position 5356, ein Adenin an Position 6762, ein Thymin an Position 7230, ein Thymin an Position 7389, ein Guanin an Position 7461, ein Adenin an Position 7536, ein Guanin an Position 8607, ein Thymin an Position 9951, ein Cytosin an Position 11460, ein Thymin an Position 11473, ein Thymin an Position 15075, ein Thymin an Position 15557, ein Adenin an Position 15773, und ein Guanin an Position 16475, wobei der Haplotyp eine erhöhte Anzahl von lebend geborenen Ferkeln anzeigt;
oder wobei der Haplotyp durch die Anwesenheit aller Polymorphismen **gekennzeichnet** ist, umfassend, verglichen mit SEQ ID Nr. 1, ein Cytosin an Position 405, ein Adenin an Position 1234, ein Cytosin an Position 3355, ein Thymin an Position 4290, ein Adenin an Position 8188, ein Thymin an Position 8466, ein Guanin an Position 9293, ein Cytosin an Position 13393, und ein Adenin an Position 16224, wobei der Haplotyp eine erhöhte Anzahl von lebend geborenen Ferkeln anzeigt;
oder wobei der Haplotyp durch die Anwesenheit aller Polymorphismen **gekennzeichnet** ist, umfassend, verglichen mit SEQ ID Nr. 1, ein Adenin an Position 124, ein Cytosin an Position 214, ein Guanin an Position 232, ein Thymin an Position 692, ein Thymin an Position 9348, und ein Cytosin an Position 15644, wobei der Haplotyp eine verringerte Anzahl von tot geborenen Ferkeln anzeigt.

2. Verfahren nach Anspruch 1, wobei der Haplotyp durch teilweises oder vollständiges Sequenzieren des mitochondriellen Genoms definiert wird.

## Revendications

1. Procédé pour l'optimisation de lignées de truies en termes d'augmentation du nombre de porcelets nés vivants par truie et/ou par an, à savoir procédé comprenant :
a) la définition des haplotypes du génome mitochondrial à partir d'un groupe de porcs,
b) la mise en corrélation desdits haplotypes avec au moins un paramètre de fertilité maternelle sélectionné à partir du groupe consistant en le nombre de porcelets nés vivants, le nombre de porcelets mort-nés, et l'intervalle de temps entre le sevrage et l'accouplement suivant d'une et unique truie, et
c) la sélection, pour l'accouplement, de la truie présentant un haplotype significativement corrélé avec au moins un paramètre de fertilité maternelle recherché sélectionné à partir du groupe consistant en une augmentation du nombre de porcelets nés vivants, une diminution du nombre de porcelets mort-nés et une réduction de l'intervalle de temps entre deux portées d'une et unique truie,
dans lequel ledit haplotype est **caractérisé par** la présence de tous les polymorphismes comprenant, par rapport à la séquence SEQ ID n° 1, une cytosine à la position 560, une thymine à la position 1089, une thymine à la position 5314, une cytosine à la position 5356, une adénine à la position 6762, une thymine à la position 7230, une thymine à la position 7389, une guanine à la position 7461, une adénine à la position 7536, une guanine à la position 8607, une thymine à la position 9951, une cytosine à la position 11460, une thymine à la position 11473, une thymine à la position 15075, une thymine à la position 15557, une adénine à la position 15773 et une guanine à la position 16475, dans lequel ledit haplotype est révélateur d'une augmentation du nombre de porcelets nés vivants ;
ou dans lequel ledit haplotype est **caractérisé par** la présence de tous les polymorphismes comprenant, par rapport à la séquence SEQ ID n° 1, une cytosine à la position 405, une adénine à la position 1234, une cytosine à la position 3355, une thymine à la position 4290, une adénine à la position 8188, une thymine à la position 8466, une guanine à la position 9293, une cytosine à la position 13393, et une adénine à la position 16224, dans lequel ledit haplotype est révélateur d'une augmentation du nombre de porcelets nés vivants ;
ou dans lequel ledit haplotype est **caractérisé par** la présence de tous les polymorphismes comprenant, par rapport à la séquence SEQ ID n° 1, une adénine à la position 124, une cytosine à la position 214, une guanine à la position 232, une thymine à la position 692, une thymine à la position 9348, et une cytosine à la position 15644, dans lequel ledit haplotype est révélateur d'une diminution du nombre de porcelets mort-nés.

2. Procédé conforme à la revendication 1, dans lequel ledit haplotype est défini par le séquençage partiel ou complet du génome mitochondrial.
